# EUROPEAN PATENT SPECIFICATION

(11) **EP 2 708 215 B1**
(45) Date of publication and mention of the grant of the patent: **08.01.2020**
(21) Application number: 12181884.3
(22) Date of filing: 27.08.2012
(51) Int. Cl.: A61F 13/14, A61B 17/132, A61B 17/135

(54) **A pressure dressing, especially for preventing hemorrhagic complications after implanting medical devices used in cardiac electrotherapy**
Druckverband, insbesondere zur Prävention von Blutungskomplikationen nach dem Implantieren von medizinischen Vorrichtungen bei der Herz-Elektrotherapie
Pansement sous pression, notamment pour la prévention de complications hémorragiques après l'implantation de dispositifs médicaux utilisés en d'électrothérapie cardiaque

(30) Priority: 13.07.2012 PL 39995612
(43) Date of publication of application: 19.03.2014
(73) Proprietor: MediNice Spolka Akcyjna, 00-805 Warszawa (PL)
(72) Inventor: LABUS, Michal, 53-650 Wroclaw (PL); SPIKOWSKI, Jerzy, 46-100 Namyslów (PL); STEC, Sebastian, 01-494 Warszawa (PL); CHOUDHARY, Sanjeev, 02-202 Warszawa (PL)
(74) Representative: AOMB Polska Sp. z.o.o.

(56) References cited:
- WO-A1-2006/069548
- WO-A1-2012/004056
- AT-B- 396 649
- US-A- 2 014 991
- US-A- 5 779 657
- US-A1- 2007 191 881
- US-A1- 2011 125 242
- Anonymous: "PaceBrace - Arm and Shoulder Restrictor for Device Pocket Implants", , 2 February 2011 (2011-02-02), XP055362083, portland oregon US Retrieved from the Internet: URL:http://www.pacebrace.com/ [retrieved on 2017-04-05]

## Description

The present invention relates to a pressure dressing, especially for preventing hemorrhagic complications in patients after surgically implanting medical devices used in cardiac electrotherapy, such as e.g. single chamber pacemakers (all types), double chamber pacemakers (all types), single and dual chamber cardioverter-defibrillators, cardioverter-defibrillators with resynchronization function and loop recorders, with special consideration of patients requiring application of a double anti-platelet therapy, heparins or vitamin K antagonists during a perioperative period.

An artificial heart pacemaker (in other words: cardiac stimulator) is an electric device implantable into a patient's body to electrically stimulate a heart rhythm. A device stimulating the heart comprises a battery-operated pacemaker, which pacemaker is implanted under the skin. A place in which a pacemaker together with battery is located is called a pacemaker pocket. From this place an electrode for stimulating the heart is led through the vein to the heart atrium or ventricle.

In patients after surgically implanting the above mentioned medical devices so called pocket hematomas may arise in the place of implementation of the device under the skin.

Arm and Shoulder Restrictors for Implants are known from "PaceBrace - Arm and Shoulder Restrictor for Device Pocket Implants", 2 February 2011, Portland Oregon US, URL:http:// www.pacebrace.com/; AT396649B and US2011125242 A1.

A pressure dressing is used to stop or to prevent bleeding after surgically implanting such medical devices, which pressure dressing serves to make a constant pressure on a desired area.

In the prior art a device for applying a pressure to a pocket for preventing hematomas after surgically implanting a medical device in the pocket, and a method of using this device, are known from US5779657 A, WO2006069548 A1, and US 2008319473 (A1). Such a device may comprise cushions and appropriate attaching means, e.g. in the form of a clamp, hinge, clasp, clip, spring, pneumatic or inflatable device, band and the like. However, this device is not provided with an automatic control and pumping system for programming assigned pressure values, responsible for maintaining a constant force of pressure.

The objective of an automated pneumatic pressure dressing is to limit a risk of hemorrhagic complications in patients after surgically implanting devices used in cardiac electrotherapy, i.e. any type of heart pacemakers (PM), cardioverter-defibrillators (ICD) and cardioverter-defibrillators with resynchronization function (ICD-CRT).

This objective is achieved according to the invention by a pressure dressing according to claim 1.

The dressing, thanks to use of a pneumatic cushion, allowing to obtain a non-traumatic pressure through a mechanical compression of a pacemaker pocket, indirectly affects a pressure on tiny blood vessels, limitation of tissue bleeding and reduction of a space within the pacemaker pocket which space could be a potential place of accumulation of blood and serous fluid. Additionally, it is characterized by a possibility of full adjustment of the force of pressure through a change of a rate of filling the pressure cushion with air, and full automation and monitoring of device's operation parameters.

Advantages of an automated pneumatic pressure dressing according to the present invention include:
- possibilities of full fitting the pressing to patient's anatomy and the place of implantation of a stimulating system;
- limitation of movability of the upper extremity on the side of the stimulating system implantation, especially abduction movement, which effectively substitutes for necessity of wearing a triangular bandage after a surgery;
- non-traumatic character of the pressure, thanks to use of a direct pressure through an air cuff of the pressure cushion;
- full automation of the device - meaning no necessity of patient's interference in functioning of the device. Thanks to use of a control and pumping module, the dressing itself watches over the proper force of pressure, automatically adjusting the pressure value in the cuff of the pressure cushion, alarming with a sound signal about necessity of reporting to an Implanting Centre, in the case of finding a leakiness of a pneumatic system or a low level of battery charge;
- monitoring of the force of pressure - the device is provided with a cache memory with the possibility of storing up to 120 measurement values of pressure in the cuff of the pressure cushion, which is a basis for automatically calculating its mean value.
- comfort and hygiene of usage - thanks to use of a set of protective covers and use of certified materials for manufacturing the dressing, as well as a disposable character of the device;
- ease and simplicity of usage - possibility of putting on the dressing directly after surgically implanting a stimulating system, in an operating room yet. Functioning of the device has an automatic character, direct participation of the patient is not required. A person having the dressing put on should be shortly instructed about the reason for putting on the dressing and the mechanism of its functioning, therein a possibility of automatic activation of a pumping mechanism, which is the sign of the correct functioning of the device.
- alarm signal - the device is provided with a sound alarm informing about impossibility of obtaining the proper pressure value in the cuff of the pressure cushion, which may indicate a leakiness of the pneumatic system or a low level of battery charge. Starting alarm should result in prompt patient's reporting to a centre implanting the pacemaker.

### The embodiment of the invention

Now, the invention will be closer presented with reference to the attached drawings, in which:

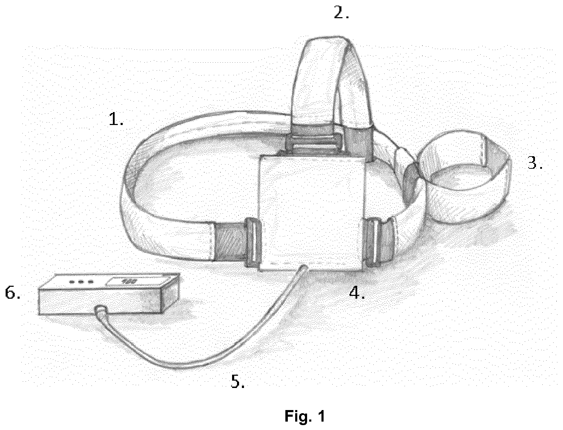

Fig. 1 is a front view of a pressure dressing according to the invention, and

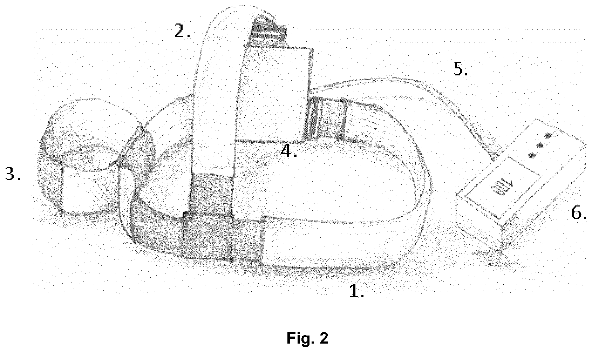

Fig. 2 is a rear view of a pressure dressing according to the invention.
The following designations are used in Fig. 1 and Fig. 2: where 1 - a chest harness belt; 2 - a shoulder harness belt; 3 - an arm harness belt; 4 - a pressure cushion; 5 - a pressure conduit; 6 - a control and pumping module with a display.

The pressure dressing according to the invention comprises carrying elements, elements of a pneumatic system and additional elements.

The carrying elements comprise a chest harness belt 1, a shoulder harness belt 2 and an arm harness belt 3, whereby elements of the harness belt (the chest harness belt 1 and the shoulder harness belt 2) allow an arbitrary fitting a pressure location to the patient's anatomy and the place of implantation of a stimulating system, and the purpose of the arm harness belt 3 is to limit movability of the upper extremity within the shoulder joint on the side of the stimulating system implantation (especially abduction movement of the upper extremity).

The elements of the pneumatic system include a pressure cushion 4, a pressure conduit 5 and a control and pumping module 6 with a display. The pressure cushion 4 in this system is an element which makes a direct pressure in the place of implantation of the stimulating system, whereas use of an air cuff in this structure allows, through coating, a very good fitting of the pressure cushion to the shape of a box of the stimulating system, which provides for a non-traumatic character of the pressure. The pressure conduit 5 connects the air cuff of the pressure cushion with the control and pumping module 6. The control and pumping module 6 with the display comprises, in turn, a controller with the display, maintaining a constant, programmed pressure value in the cuff of the pressure cushion 4, and an electric pumping system, which is automatically switched on in the case when pressure in the cuff of the pressure cushion 4 falls below the programmed value. Use of a pressure sensor and the electric pumping system operating automatically (according to a programmed algorithm) provides for maintaining a constant value of the force of pressure expressed with the pressure value measured in the cuff of the pressure cushion 4 (without patient's interference). Additionally, the system is provided with a liquid crystal screen, on which current pressure in the cuff of the pressure cushion and current time is displayed. Moreover, the controller has its own memory, in which the pressure values in the cuff of the pressure cushion 4 are stored every 60 minutes, on the basis of which the mean value of the force of pressure, expressed in millimetres of mercury, is calculated, which allows a monitoring of correct functioning of the device.

The control module includes also an alarm function, which with a sound signal informs the patient about necessity of reporting to a Centre implanting the system, in the case of a leakiness of the pneumatic system or a low level of battery charge.

Elements of a protective cover being the additional element, are suitably matched up to the above mentioned elements of the present dressing, providing for comfort and hygiene of usage of the pressure dressing according to the invention.

The dressing according to the present invention is provided with the pressure cushion 4, which through a mechanical pressure on the pacemaker box and surrounding tissues serves the aim of minimizing a local bleeding, by which it prevents arising hematomas of a pocket in the place of implanting the stimulating system. Thanks to use of the air cuff, a possibility of full adjustment of the force of pressure, dependent on a rate of filling of the cuff, is obtained. Use of an integrated circuit with the pressure sensor and an electric pump in the structure of the control and pumping unit has allowed obtaining a fully automated, dependent on the programmed algorithm, control of the rate of filling of the cuff of the pressure cushion. Additionally, the system is provided with the cache memory, in which the measurements of pressure value in the cuff of the pressure cushion with a specific time intervals are stored, and the sound alarm informing about a possibility of a leakiness of the pneumatic system or a low level of battery charge. A suitably selected system of the carrying belts provides for possibility of a very good fitting of the dressing to anatomy and the place of implantation of the stimulating system, and is also responsible for a desired limitation of movability of the upper extremity within the shoulder joint after surgically implanting.

A pressure dressing according to the present invention is applicable in prevention of pocket hematomas after implanting heart stimulating systems, such as:
- single chamber pacemakers (all types);
- double chamber pacemakers (all types);
- single and dual chamber cardioverter-defibrillators;
- cardioverter-defibrillators with resynchronization function;
- loop recorders;
with special consideration of patients requiring application of a double anti-platelet therapy, heparins or vitamin K antagonists during a perioperative period.

## Claims

1. A pressure dressing for preventing hemorrhagic complications after implanting medical devices in cardiac electrotherapy, comprising attaching means constituting a harness and having a chest belt (1) and a shoulder belt (2) connected to a pneumatic pressure cushion (4),
the pressure dressing comprises a pressure sensor connected with an electric control and pumping system (6) provided with an integrated circuit for controlling pressure in the pressure cushion (4), wherein
the harness comprises also an arm belt (3),
the integrated circuit is programmed so as to maintain an assigned constant pressure in the pressure cushion (4),
the control and pumping system (6) has a display, preferably a liquid crystal screen, capable of displaying a value of current pressure in a cuff of the pressure cushion (4) or current time, **characterized in that**
the control and pumping system (6) has a controller having a memory,
in which the pressure values in the cuff of the pressure cushion 4 are stored in periods from 1 to 360 minutes, preferably from 30 to 120 minutes, even more preferably every 60 minutes, on the basis of which the mean value of the force of pressure, expressed in millimeters of mercury, is calculated, the control and pumping system (6) is battery-operated,
the pressure dressing further includes an alarm, preferably a sound alarm, warning about a leakiness of the pneumatic system or about a low level of battery charge.

## Patentansprüche

1. Druckverband zur Verhinderung von hämorrhagischen Komplikationen nach der Implantieren von medizinischen Vorrichtungen bei der Herzelektrotherapie, umfassend ein Befestigungsmittel, das ein Geschirr darstellt und einen Brustgurt (1) und einen Schultergurt (2) aufweist, der mit einem pneumatischen Druckkissen (4) verbunden ist,
der Druckverband einen Drucksensor umfasst, der mit einem elektrischen Steuer- und Pumpsystem (6) verbunden ist, das mit einer integrierten Schaltung zum Steuern des Drucks in dem Druckkissen (4) versehen ist, wobei
das Geschirr auch einen Armgurt (3) umfasst,
die integrierte Schaltung so programmiert ist, um im Druckkissen (4) ein zugeordneter konstanter Druck aufrechtzuerhalten,
das Steuer- und Pumpsystem (6) eine Anzeige hat, vorzugsweise einen Flüssigkristallbildschirm, die einen Wert des aktuellen Drucks in einer Manschette des Druckkissens (4) oder der aktuellen Zeit anzeigen kann, **dadurch gekennzeichnet, dass** das Steuer- und Pumpsystem (6) eine Steuerung mit einem Speicher aufweist,
bei dem die Druckwerte in der Manschette des Druckkissens (4) in Zeiträumen von 1 bis 360 Minuten, bevorzugt von 30 bis 120 Minuten, besonders bevorzugt alle 60 Minuten gespeichert werden, auf deren Grundlage der Mittelwert der Druckkraft, in Millimeter Quecksilbersäule ausgedrückt, berechnet wird,
das Steuer- und Pumpsystem (6) batteriebetrieben ist,
der Druckverband ferner ein Alarmgerät, vorzugsweise ein akustisches Alarmgerät enthält, der vor einer Undichtigkeit des pneumatischen Systems oder einem niedrigen Ladezustand der Batterie warnt.

## Revendications

1. Un pansement de pression pour prévenir les complications hémorragiques après implantation de dispositifs médicaux en électrothérapie cardiaque, comprenant des moyens de fixation constituant un harnais et comportant une ceinture thoracique (1) et une ceinture d'épaule (2) reliées à un coussin de pression pneumatique (4),
le pansement de pression comprend un capteur de pression relié à un système électrique de commande et de pompage (6) pourvu d'un circuit intégré pour contrôler la pression dans le coussin de pression (4), dans lequel
le harnais comprend également une ceinture de bras (3),
le circuit intégré est programmé de manière à maintenir une pression constante assignée dans le coussin de pression (4),
le système de commande et de pompage (6) comporte un affichage, de préférence un écran à cristaux liquides, capable d'afficher une valeur de la pression actuelle dans un brassard du coussin de pression (4) ou de l'heure actuelle, **caractérisé en ce que**
le système de commande et de pompage (6) comporte un contrôleur ayant une mémoire, dans laquelle les valeurs de pression dans le brassard du coussin de pression 4 sont stockées dans des périodes de 1 à 360 minutes, de préférence de 30 à 120 minutes, de manière encore plus préférée toutes les 60 minutes, sur la base desquelles la valeur moyenne de la force de pression, exprimé en millimètres de mercure, est calculée,
le système de commande et de pompage (6) fonctionne sur accumulateurs,
le pansement de pression comprend en outre une alarme, de préférence une alarme sonore, avertissant d'une fuite du système pneumatique ou d'un faible niveau de charge des accumulateurs.
